# FASCICULE DE BREVET EUROPEEN

(11) **EP 0 909 554 B1**
(45) Date de publication et mention de la délivrance du brevet: **22.01.2003**
(21) Numéro de dépôt: 98420182.2
(22) Date de dépôt: 09.10.1998
(51) Int. Cl.: A61F 2/18

(54) **Prothèse d'oreille moyenne**
Mittelohrprothese
Middle ear prosthesis

(30) Priorité: 10.10.1997 FR 9712995
(43) Date de publication de la demande: 21.04.1999
(73) Titulaire: Nogitek, 35760 Saint Gregoire (FR)
(72) Inventeur: Magnan, Jacques, 13360 Roquevaire (FR); Giquel, Loic, 35800 Dinard (FR); Prandi, Bernard, 74210 Seythenex (FR)
(74) Mandataire: Myon, Gérard Jean-Pierre

(56) Documents cités:
- EP-A- 0 379 740
- WO-A-92/18066
- FR-A- 2 691 354
- SU-A- 1 634 272
- US-A- 4 957 507

## Description

La présente invention a trait à une prothèse ossiculaire de l'oreille moyenne, du type destinée à remplacer au moins partiellement la chaîne ossiculaire et à relier un premier organe, notamment la platine de l'étrier ou l'étrier, avec un second organe, notamment le marteau ou le tympan, ladite prothèse étant apte à s'auto-ajuster par élasticité.

L'oreille moyenne est destinée à transmettre les vibrations sonores atteignant le tympan depuis un milieu aérien, jusqu'à un milieu liquidien, à savoir l'oreille interne, qui constitue un organe de perception récepteur d'une onde sonore.

La conduction de cette dernière s'effectue au travers d'un élément anatomique appelé système tympano-ossiculaire, composé d'une membrane tympanique attenante à une chaîne ossiculaire formée, de dehors en dedans, du marteau, de l'enclume et de l'étrier. La membrane tympanique est attenante à cette chaîne par l'intermédiaire du manche du marteau. L'étrier repose sur une platine qui entre en contact, par sa base interne, avec le milieu liquidien, dénommé encore milieu labyrinthique, de l'oreille interne.

La fonction des osselets de la chaîne ossiculaire est d'assurer le contact entre le tympan et ce milieu labyrinthique, ce qui constitue l'effet columellaire. Le système tympano-ossiculaire est suspendu dans la cavité aérienne de l'oreille moyenne. Il n'existe donc pas de contact direct de ce système avec les parois osseuses, afin de véhiculer l'intégralité de l'énergie acoustique provenant de la membrane tympanique, à savoir de l'oreille externe, en direction de l'oreille interne.

Le système tympano-ossiculaire a pour point de fixation deux ligaments annulaires disposés à ses extrémités, à savoir celui de la membrane tympanique et celui de la platine de l'étrier.

L'invention a pour objet la correction des surdités de transmission, à savoir celles résultant d'atteintes de la chaîne ossiculaire à la suite de différentes pathologies.

Les prothèses ossiculaires de l'oreille moyenne sont destinées à remplacer au moins partiellement cette chaîne ossiculaire, à savoir soit uniquement l'enclume, soit l'étrier et l'enclume, ou bien encore l'étrier, l'enclume et le marteau.

On connaît de telles prothèses qui sont réalisées en différents matériaux tels que la céramique, et se composent d'une tige reposant sur l'étrier ou sur sa platine, surmontée par un plateau qui repose sous le manche du marteau ou le tympan. Ces prothèses présentent une structure essentiellement rigide, du fait de la nature de leur matériau constitutif.

Cette solution présente un certain nombre d'inconvénients. Ainsi, il est tout d'abord nécessaire que le chirurgien ait à sa disposition un grande nombre de prothèses de tailles et de géométries différentes, afin de pouvoir choisir celle qui est la mieux adaptée à l'implantation qu'il doit effectuer.

De plus, il est nécessaire de modifier la taille de ce type de prothèses, soit en les sectionnant, soit en les fraisant, en vue d'une adéquation de leurs dimensions avec celles des "défects", à savoir des éléments absents de la chaîne ossiculaire. Ceci entraîne une modification de l'état de surface de la prothèse, ce qui contribue à la fragiliser, modifie localement son interface et constitue un point d'appel à la réaction inflammatoire.

Enfin, étant donné que ces prothèses sont rigides et présentent des dimensions qui ne peuvent être modifiées, leur point d'ancrage sur les organes qu'elles sont destinées à relier consiste en un simple contact, de sorte qu'il s'opère uniquement une mise en tension passive de la prothèse entre ces organes. Ceci ne permet pas la reconstitution d'un ensemble parfaitement stable, même à la suite d'une parfaite mise en place, ce qui explique que de nombreux déplacements postopératoires sont constatés. Ainsi, seulement environ 75% des prothèses partielles et 50% des prothèses totales se sont révélées présenter une tenue mécanique suffisante.

Afin de rémédier à ces problèmes de stabilité, il a été proposé d'autres prothèses d'oreille moyenne, notamment par WO-A-92/18066. Ce document décrit une prothèse composée de deux pièces indépendantes maintenues en contact et mobiles l'une par rapport à l'autre au moyen d'un système mécanique à ressort. Une telle conformation assure une possibilité de déformation de la prothèse durant sa mise en place. Cependant, les contraintes mécaniques exercées par ces prothèses sur les organes qu'elles relient ne sont pas constantes du fait des forces de frottement intervenant entre les deux éléments constitutifs de la prothèse. Ceci induit un risque important de traumatisme au niveau des surfaces des organes en contact avec cette prothèse. De plus, cette dernière présente une structure mécanique relativement complexe induisant donc un coût élevé et dont le fonctionnement peut être altéré par la présence de tissus au niveau du ressort.

FR-A-2 691 354 montre une prothèse selon le préambule de la revendication 1.

L'invention se propose de pallier les inconvénients de l'art antérieur évoqués ci-dessus.

A cet effet, elle a pour objet une prothèse d'oreille moyenne du type connu du FR-A-2 691 354 caractérisée par les caractéristiques de la partie caractérisant de la revendication 1.

Le terme d'élément allongé désigne notamment un élément formé au moins partiellement par une lamelle mince.

La prothèse conforme à l'invention permet de réaliser les objectifs précédemment mentionnés. Ainsi, elle présente une structure mécanique extrêmement simple, puisqu'elle est formée par un élément unique qui ne doit être ni taillé ni fraisé durant sa pose, ce qui permet de réduire le risque de réaction inflammatoire et les phénomènes de lyses.

De plus, étant donné que les ailes de la partie cintrée sont aptes à se rapprocher et à s'éloigner mutuellement par élasticité, il existe une parfaite adaptation entre les dimensions de la prothèse et celles séparant les organes qu'elle doit relier. La nature élastique de cette partie cintrée lui permet donc de s'ajuster, non seulement en fonction des différentes tailles rencontrées selon les patients, mais aussi en fonction des vibrations exercées par la fibrose post-opératoire au niveau de la surface du tympan. Ceci garantit donc une stabilité très satisfaisante de la prothèse conforme à l'invention.

En outre, cette partie cintrée présente une flexibilité suffisante, de sorte qu'elle peut être mise en place sans tension excessive lors de sa pose, et qu'elle peut exercer, une fois posée, une tension permanente à peu près constante au niveau de ses points d'ancrage respectifs.

Selon un premier mode de réalisation de l'invention, la partie cintrée est destinée à entrer en contact avec les premier et second organes, par la surface extérieure de ses deux ailes. Dans ce mode de réalisation, la prothèse conforme à l'invention constitue une prothèse d'enclume permettant de relier le marteau et l'étrier.

Selon une caractéristique supplémentaire de l'invention, une encoche destinée à recevoir le bouton de l'étrier est ménagée dans la seconde aile de la portion cintrée. Ceci garantit un ancrage satisfaisant sur le long terme de l'ensemble de la prothèse avec l'étrier.

Selon un second mode de réalisation de l'invention, la portion cintrée est destinée en entrer en contact avec un des organes par la surface extérieure d'une première aile, et elle est prolongée, au niveau d'une seconde aile, par une portion sensiblement rectiligne. Cette portion sensiblement rectiligne permet à la prothèse conforme à l'invention de prendre appui sur la platine de l'étrier, de sorte qu'elle est à même de constituer une prothèse totale d'enclume et d'étrier ou bien encore de marteau, d'enclume et d'étrier. Cette portion rectiligne assume en outre une fonction de transmission des vibrations sonores, jusqu'à la platine de l'étrier. Dans le cas d'une prothèse partielle, la portion rectiligne est disposée sensiblement dans le prolongement de la seconde aile alors que, pour une prothèse totale, la portion rectiligne est à peu près perpendiculaire à cette seconde aile.

Selon une caractéristique supplémentaire de l'invention, la portion rectiligne est prolongée, à son extrémité opposée à la portion cintrée, par une portion coudée. Cette dernière prend appui sur la platine de l'étrier, et garantit une bonne stabilité de la prothèse par rapport à cette dernière.

Selon une caractéristique supplémentaire de l'invention, la portion cintrée est prolongée, au niveau de sa première aile, par une portion galbée dont la concavité est dirigée à l'opposé de la portion rectiligne. Cette portion galbée assure un maintien satisfaisant de la prothèse sur le premier organe qu'elle est destinée à relier, à savoir le tympan ou le marteau.

Selon une caractéristique avantageuse de l'invention, la prothèse est réalisée en un alliage métallique superélastique à la température du corps. On entend par alliage superélastique tout alliage dont le domaine d'élasticité est plus vaste que celui des métaux classiques, et pour lequel la force exercée sur le palier d'élasticité est pratiquement constante.

Selon une caractéristique préférée de l'invention, l'alliage superélastique est un alliage à mémoire de forme.

Un alliage à mémoire de forme présente, de part et d'autre d'une température de transition, une structure cristalline austénitique stable à chaud et une structure martensitique stable à froid. En outre, lorsqu'une pièce réalisée en un alliage à mémoire de forme subit une contrainte, dans sa structure austénitique, elle voit sa structure devenir au moins partiellement martensitique, ce qui conduit à une déformation élastique très importante pouvant atteindre 8%.

L'utilisation d'alliages métalliques superélastiques et, en particulier, d'alliages à mémoire de forme est particulièrement avantageuse. En effet, la partie cintrée absorbe seule l'ensemble des contraintes, en cours de pose par ajustement de ses dimensions. De plus, cette partie cintrée adapte sa géométrie en fonction de la taille du défaut ou "défect", à savoir des éléments de la chaîne ossiculaire qu'elle est destinée à remplacer. Du fait de sa nature superélastique, cette partie cintrée exerce sur le tympan une force très faible et sensiblement constante, ce qui est particulièrement avantageux à l'égard de l'intégrité physiologique du tympan et de la platine.

Une fois la prothèse en place, sa partie rectiligne présente en permanence une structure austénitique, ce qui lui permet de posséder les propriétés de rigidité nécessaires pour assurer une transmission satisfaisante des vibrations, alors que sa partie cintrée peut présenter une structure au moins partiellement martensitique, en fonction de l'importance de la déformation.

Selon une caractéristique avantageuse de l'invention, l'alliage à mémoire de forme possède une température de fin d'apparition austénitique (A_{f}) sans contrainte mécanique, qui est inférieure à 30°C et, de préférence, inférieure à 20°C.

Selon une caractéristique supplémentaire de l'invention, l'alliage à mémoire de forme se compose de titane et de nickel, et il contient 55,7% ± 0,4% de nickel.

L'invention va être décrite ci-dessous, en référence aux dessins annexés, donnés uniquement à titre d'exemples non limitatifs et dans lesquels :
- la figure 1A représente une chaîne ossiculaire incomplète, dans laquelle l'enclume est absente ;
- la figure 1B représente une première prothèse conforme à l'invention ;
- la figure 1C représente la prothèse illustrée à la figure 1B, insérée au sein de la chaîne ossiculaire de la figure 1A ;
- la figure 1D représente une chaîne ossiculaire incomplète, dans laquelle l'enclume et le marteau sont absents ;
- la figure 1E représente la prothèse illustrée à la figure 1B, insérée au sein de la chaîne ossiculaire de la figure 1D ;
- la figure 2A représente une chaîne ossiculaire incomplète, dans laquelle l'étrier et l'enclume sont absents ;
- la figure 2B illustre, en perspective, une deuxième prothèse conforme à l'invention ;
- la figure 2C illustre la prothèse représentée à la figure 2B, insérée au sein de la chaîne ossiculaire de la figure 2A ;
- la figure 3A représente, en pointillés, une chaîne ossiculaire totalement absente ;
- la figure 3B illustre une troisième prothèse conforme à l'invention, et
- la figure 3C représente la prothèse illustrée à la figure 3B et remplaçant la chaîne ossiculaire de la figure 3A.

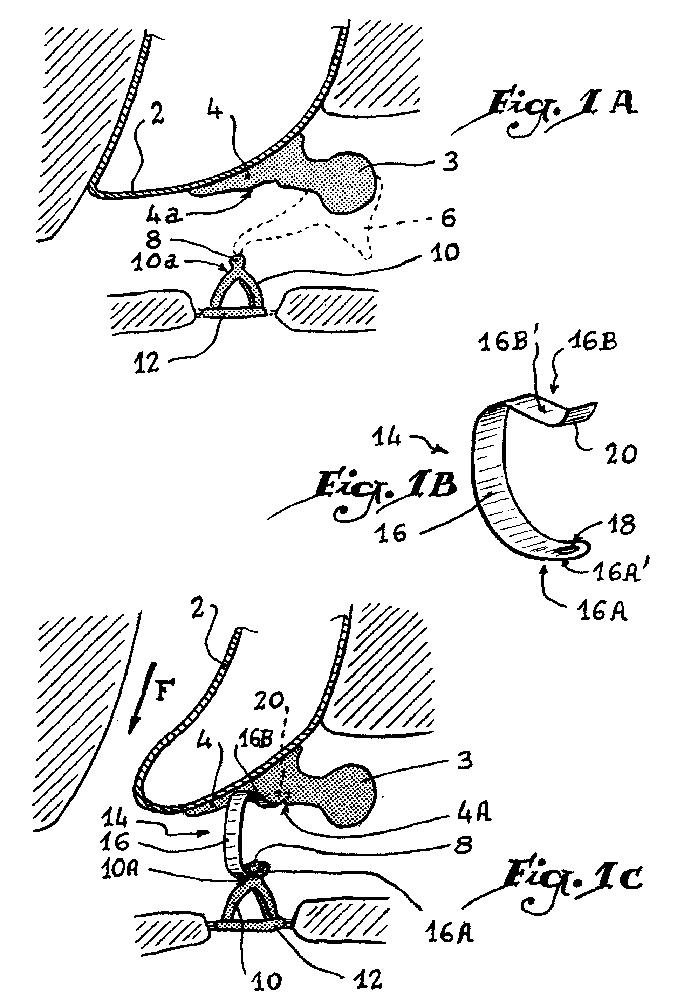

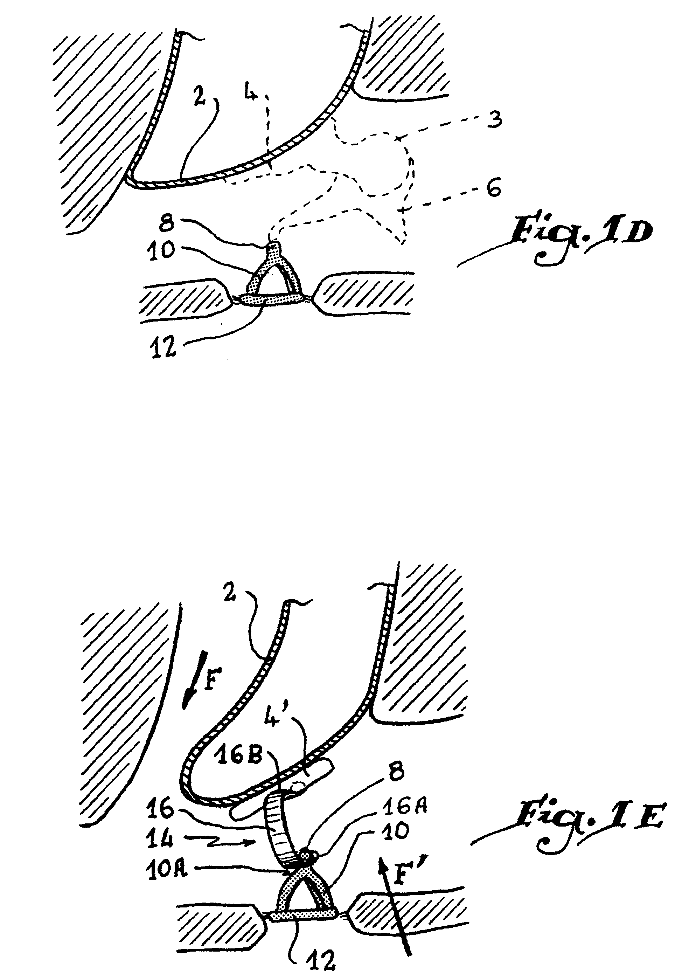

La figure 1A représente l'oreille moyenne d'un patient. Elle comprend, de dehors en dedans, un tympan 2 attenant au marteau 3 par son manche 4. La tête du marteau présente, en arrière, une surface articulaire qui est destinée à s'unir avec l'enclume 6 dont l'apophyse lenticulaire s'articule avec la tête 8 de l'étrier 10. La base interne de la platine 12 de l'étrier est dirigée vers le plancher du vestibule de l'oreille interne non représentée.

La chaîne ossiculaire, composée du marteau, de l'enclume et de l'étrier, est incomplète dans la mesure où l'enclume 6 est absente et se trouve de ce fait représentée en traits interrompus.

Dans ce qui suit, et pour plus de clarté, la tête du patient est en position opératoire, c'est-à-dire décubitus dorsal, tête tournée du côté opposé à l'oreille représentée, ici l'oreille gauche.

La figure 1B représente une prothèse désignée dans son ensemble par la référence 14 et destinée à remplacer l'enclume 6 au sein de la chaîne ossiculaire de la figure 1A.

Cette prothèse est réalisée en un alliage à mémoire de forme composé de 55,75% de nickel et de 44,25% de titane, dont la structure est superélastique à la température du corps.

Cette prothèse 14 comprend une portion cintrée 16 sensiblement en forme de C dont l'aile inférieure 16A est pourvue d'une encoche traversante 18, et l'aile supérieure 16B est prolongée par une portion galbée 20 dont la concavité est dirigée à l'opposé de l'aile inférieure 16A destinée à entrer en contact avec le manche 4 du marteau.

La distance séparant les deux ailes 16A, 16B est légèrement supérieure à celle séparant la tête 8 de l'étrier par rapport à la surface inférieure du manche 4 du marteau. De la sorte, comme le montre la figure 1C, la surface extérieure 16B' de l'aile supérieure 16B repose, une fois la prothèse montée, contre la surface inférieure 4A du manche 4. De plus, la surface extérieure 16A' de l'aile inférieure 16A entre en contact avec la surface supérieure 10A de l'étrier 10, l'encoche 18 de cette aile 16A s'insérant sur la tête 8 de l'étrier.

La pose de la prothèse conforme à l'invention s'effectue de manière connue soit par la voie du conduit auditif externe, ou voie endaurale, selon la flèche F, soit par la voie de la tympanotomie postérieure selon la flèche F'. A cet effet, le chirurgien place l'orifice 18 sur la tête 8 de l'étrier. La nature superélastique de l'alliage utilisé permet de plier la prothèse de manière à rapprocher mutuellement ses ailes 16A et 16B, ce qui permet de glisser aisément l'aile supérieure 16B sous le manche 4 du marteau.

La prothèse 14 conforme à l'invention peut également être utilisée dans le cas où le marteau est absent, la figure 1D représente la chaîne ossiculaire incomplète dans la mesure où l'enclume et le marteau sont absents et se trouvent de ce fait représentés en traits interrompus.

La pose de la prothèse 14 conforme à l'invention sur la tête 8 de l'étrier est identique à ce qui vient d'être décrit. Le marteau 4 est remplacé par un fragment cartilagineux 4' destiné à entrer en contact avec l'aile supérieure 16B de la prothèse 14.

De plus, la géométrie de la portion galbée 20 garantit une parfaite complémentarité de formes entre le manche 4 ou le fragment cartilagineux 4' et la prothèse. La nature superélastique de cette dernière lui permet de s'ajuster non seulement en fonction des tailles des défects des différents patients, mais encore en fonction des efforts exercés par l'étrier et le marteau.

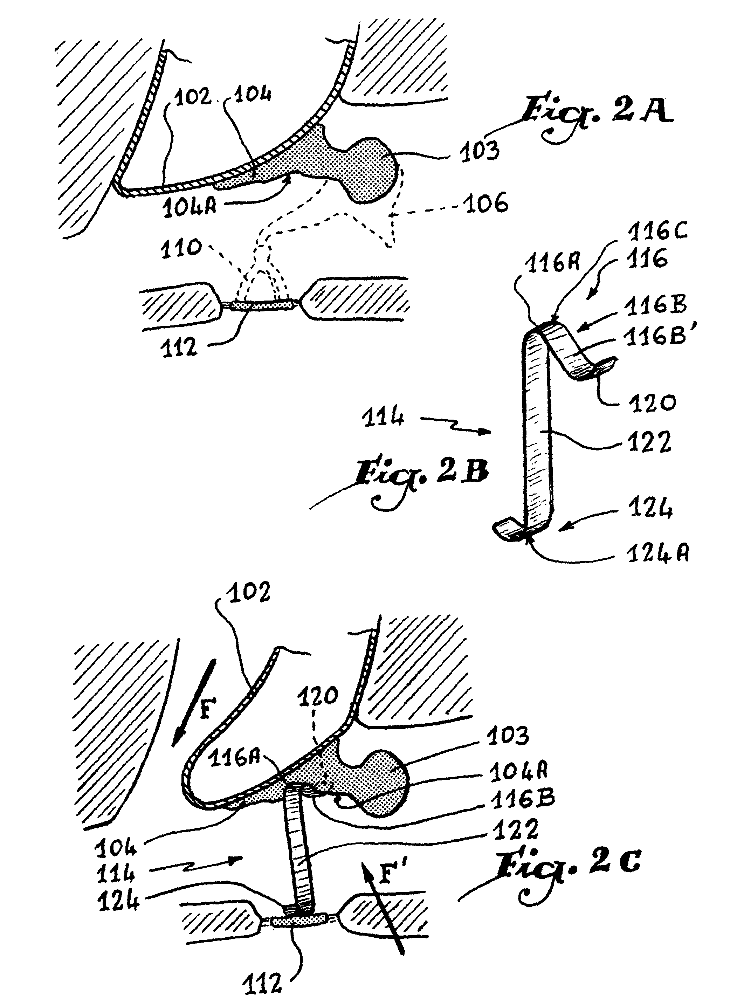

La chaîne ossiculaire représentée à la figure 2A diffère de celle illustrée à la figure 1A en ce que non seulement l'enclume 106, mais également l'arche de l'étrier 110 sont absents et de ce fait représentés en traits interrompus. La prothèse 114, représentée à la figure 2B et destinée à remplacer ces deux organes, comprend une portion cintrée 116 sensiblement en forme de C, dont une première aile 116B, ou aile distale est prolongée par une portion galbée 120 sensiblement analogue à celle 20 illustrée aux figures 1B et 1C. La seconde aile 116A de cette portion cintrée 116, ou aile proximale se poursuit par une portion sensiblement rectiligne 122 orientée selon le prolongement de cette aile inférieure 116A. Cette portion rectiligne 122 est terminée par une portion coudée 124 faisant saillie en direction de l'âme 116C de la portion cintrée, sensiblement selon un angle droit à partir de la portion rectiligne 122.

La distance séparant la surface extérieure 116B' de l'aile distale 116B, et la surface inférieure 124A de la portion coudée 124, est légèrement supérieure à la distance séparant la platine 112 et la surface inférieure 104A du manche 104 du marteau 103.

Le montage de la prothèse 114 s'effectue en plaçant la surface inférieure 124A de la portion coudée 124 sur la platine 112, puis en rapprochant l'aile distale 116B de la portion cintrée 116 en direction de l'aile proximale 116A, grâce à la nature du matériau superélastique utilisé.

Il est alors aisé d'insérer cette aile proximale 116A sous le manche 104 du marteau 103. Cette opération de pose est simple et permet de ne pas endommager les éléments ossiculaire destinés à entrer en contact avec la prothèse.

Etant donné que la portion cintrée 116 est soumise aux contraintes exercées par le marteau et la platine de l'étrier, elle présente une structure au moins partiellement martensitiques. Ses ailes 116A, 116B peuvent donc s'ajuster en fonction de la taille des chaînes ossiculaires des différents patients et des efforts exercés sur le marteau et la platine 112 de l'étrier lors du méchage ou de la fibrose post-opératoire, ce qui confère une excellente stabilité à la prothèse.

La portion rectiligne 122 présente, quant à elle, une structure austénitique, ce qui lui confère une rigidité lui permettant de transmettre les vibrations sonores depuis le tympan 102 jusqu'au milieu labyrinthique de l'oreille interne.

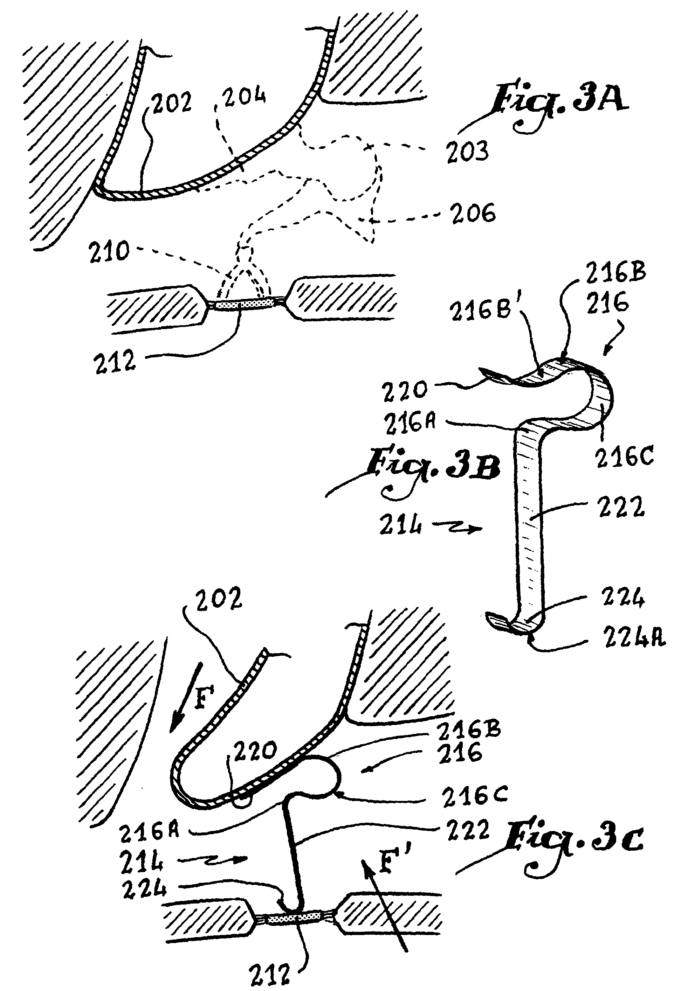

L'oreille moyenne représentée à la figure 3A est totalement dépourvue de chaîne ossiculaire, puisqu'à la fois le marteau 203, l'enclume 206 et l'ensemble de la structure de l'étrier 210 sont absents, et sont de ce fait représentés en traits interrompus.

La prothèse 214 est destinée à remplacer l'ensemble de cette chaîne ossiculaire. Elle comprend une portion cintrée 216 sensiblement en forme de C, dont l'aile supérieure 216B se termine par une portion galbée 220 dont la concavité est dirigée à l'opposé de l'aile inférieure 216A de la portion cintrée. Cette aile inférieure 216A se prolonge par une portion rectiligne 222 se raccordant à la portion cintrée, sensiblement selon un angle droit. Cette portion rectiligne 222 est terminée par une portion coudée 224 opérant un décrochement sensiblement selon un angle droit, et faisant saillie en direction opposée à l'âme 216C de la portion cintrée.

La distance séparant la surface extérieure 216B' de l'aile supérieure 216B, et la surface inférieure 224A de la portion coudée 224, est légèrement plus importante que celle séparant le tympan 202 de la platine 212.

Le montage de la prothèse 214 s'opère de manière analogue à ce qui a été décrit aux figures 2A à 2C, soit selon la flèche F par la voie du conduit auditif externe, soit selon la flèche F' par la voie de la tympanotomie postérieure. Le chirurgien rapproche les ailes 216A, 216B en tirant parti de la nature superélastique de l'alliage constitutif de la prothèse, ce qui permet à cette dernière d'être insérée sans endommager ni la platine, ni le tympan.

Une fois la prothèse montée, elle prend appui contre le tympan 202, à la fois par son aile supérieure 216B et sa portion galbée 220 dont la géométrie assure une parfaite complémentarité de formes avec le tympan. La prothèse repose, par la surface inférieure 224A de sa portion coudée 224, sur la platine 212 de l'étrier.

La portion cintrée 216 est soumise aux efforts exercés par le tympan et la platine de l'étrier et possède une structure austénitique ou au moins partiellement martensitique, en fonction de l'intensité de ces efforts. Les dimensions de cette portion cintrée 216 sont alors susceptibles de s'ajuster à la fois en fonction de la taille des différents défects ossiculaires des patients, et des efforts qu'exercent sur cette portion à la fois le tympan et la platine de l'étrier. Il est à noter que, du fait de sa nature superélastique, les forces exercées en réaction, sur le tympan et la platine de l'étrier, respectivement par la portion cintrée et par la portion galbée, sont très faibles et sensiblement constantes. Ceci est particulièrement avantageux pour préserver l'intégrité physiologique du tympan et de la platine, et pour assurer une stabilité satisfaisante de la chaîne ossiculaire ainsi reconstruite.

De plus, la portion rectiligne 222, qui présente une structure austénitique, est à même d'assumer de manière satisfaisante une fonction de transmission des vibrations sonores grâce à ses propriétés de rigidité.

## Revendications

1. Prothèse (14 ; 114 ; 214) ossiculaire de l'oreille moyenne, du type destinée à remplacer au moins partiellement la chaîne ossiculaire (3, 6, 10 ; 103, 106, 110 ; 203, 206, 210) et à relier un premier organe, notamment la platine de l'étrier ou l'étrier, avec un second organe, notamment le marteau ou le tympan, ladite prothèse étant apte à s'auto-ajuster par élasticité, ladite prothèse étant formée par un élément allongé qui comprend une portion cintrée (16 ; 116 ; 216) présentant sensiblement une forme de C, dont les ailes sont aptes à se rapprocher et à s'éloigner mutuellement par élasticité, **caractérisée en ce que** ladite portion cintrée est destinée à entrer en contact avec au moins un desdits organes par une surface extérieure (16A', 16B' ; 116B' ; 216B') d'au moins une (16A, 16B ; 116B ; 216B) de ses ailes (16A, 16B ; 116A, 116B ; 216A, 216B).

2. Prothèse selon la revendication 1, **caractérisée en ce que** ladite portion cintrée (16) est destinée à entrer en contact avec lesdits premier et second organes par la surface extérieure (16A', 16B') de ses deux ailes (16A, 16B).

3. Prothèse selon la revendication 1 ou 2, **caractérisée en ce qu'**une encoche (18) destinée à recevoir le bouton (8) de l'étrier (10), est ménagée dans ladite seconde aile (16A) de ladite portion cintrée (16).

4. Prothèse selon la revendication 1, **caractérisée en ce que** ladite portion cintrée (116 ; 216) est destinée à entrer en contact avec un desdits organes par la surface extérieure (116B', 216B') d'une première aile (116B ; 216B) et est prolongée, au niveau d'une seconde aile (116A ; 216A), par une portion sensiblement rectiligne (122 ; 222).

5. Prothèse selon la revendication 4, **caractérisée en ce que** ladite portion rectiligne (122 ; 222) est prolongée, à son extrémité opposée à la portion cintrée (116 ; 216), par une portion coudée (124 ; 224) destinée à être supportée par la platine de l'étrier.

6. Prothèse selon l'une quelconque des revendications 1 à 5, **caractérisée en ce que** ladite portion cintrée (16) est prolongée, au niveau d'une première aile (16B), par une portion galbée (20) dont la concavité est dirigée à l'opposé d'une seconde aile (16A) de ladite portion cintrée (16).

7. Prothèse selon l'une des revendications 1 à 6, **caractérisée en ce qu'**elle est réalisée en un alliage métallique superélastique à la température du corps.

8. Prothèse selon la revendication 7, **caractérisée en ce que** ledit alliage superélastique est un alliage à mémoire de forme.

9. Prothèse selon la revendication 8, **caractérisée en ce que** ledit alliage possède une température de fin d'apparition austénitique (A_{f}) sans contrainte mécanique, qui est inférieure à 30°C et, de préférence, inférieure à 20°C.

10. Prothèse selon la revendication 8 ou 9, **caractérisée en ce que** ledit alliage se compose de titane et de nickel, et **en ce qu'**il contient 55,7% ± 0,4% de nickel.

## Patentansprüche

1. Gehörknöchelchenprothese (14; 114; 214) für das Mittelohr, von der Art, die dazu vorgesehen ist, mindestens teilweise die Gehörknöchelchenkette (3, 6, 10; 103, 106, 110; 203, 206, 210) zu ersetzen und ein erstes Organ, insbesondere die Fußplatte des Steigbügels oder den Steigbügel, mit einem zweiten Organ, insbesondere den Hammer oder das Trommelfell, zu verbinden, wobei die Prothese so ausgestaltet ist, dass sie sich durch Elastizität selbst anpasst, wobei die Prothese durch ein längliches Element ausgestaltet ist, das einen gewölbten Abschnitt (16; 116; 216) umfasst, der im Wesentlichen eine C-Form aufweist, dessen Flügel in der Lage sind, sich aufgrund von Elastizität einander anzunähern und voneinander weg zu bewegen, **dadurch gekennzeichnet, dass** der gewölbte Abschnitt dazu vorgesehen ist, mit mindestens einem der Organe mittels einer Außenfläche (16A', 16B'; 116B'; 216B') von mindestens einem (16A, 16B; 116B; 216B) seiner Flügel (16A, 16B; 116A, 116B; 216A, 216B) in Kontakt zu treten.

2. Prothese nach Anspruch 1 **dadurch gekennzeichnet, dass** der gewölbte Abschnitt (16) dazu vorgesehen ist, mit dem ersten und dem zweiten Organ durch die Außenfläche (16A', 16B') seiner beiden Flügel (16A, 16B) in Kontakt zu treten.

3. Prothese nach Anspruch 1 oder 2, **dadurch gekennzeichnet, dass** in dem zweiten Flügel (16A) des gewölbten Abschnittes (16) eine Ausnehmung (18) ausgestaltet ist, die dazu vorgesehen ist, den Knopf (8) des Steigbügels (10) aufzunehmen.

4. Prothese nach Anspruch 1, **dadurch gekennzeichnet, dass** der gewölbte Abschnitt (116; 216) dazu vorgesehen ist, mit einem der Organe durch die Außenfläche (116B', 216B') eines ersten Flügels (116B; 216B) in Kontakt zu treten, wobei dieser in Höhe eines zweiten Flügels (116A; 216A) durch einen im Wesentlichen geradlinigen Abschnitt (122; 222) verlängert ist.

5. Prothese nach Anspruch 4, **dadurch gekennzeichnet, dass** der geradlinige Abschnitt (122; 222) an seinem, das dem gewölbten Abschnitt (116; 216) gegenüberliegenden Ende durch einen abgewinkelten Abschnitt (124; 224) verlängert ist, der dazu vorgesehen ist, durch die Fußplatte des Steigbügels abgestützt zu werden.

6. Prothese nach irgendeinem der Ansprüche 1 bis 5, **dadurch gekennzeichnet, dass** der gewölbte Abschnitt (16), in Höhe eines ersten Flügels (16B) durch einen nach oben ausgebogenen Abschnitt (20) verlängert ist, dessen Ausbiegung zur entgegen gesetzten Seite eines zweiten Flügels (16A) des gewölbten Abschnittes (16) gerichtet ist.

7. Prothese nach einem der Ansprüche 1 bis 6. **dadurch gekennzeichnet, dass** sie aus einer metallischen Legierung, die bei Körpertemperatur hochelastisch ist, hergestellt ist.

8. Prothese nach Anspruch 7, **dadurch gekennzeichnet, dass** die hochelastische Legierung eine Formgedächtnislegierung ist.

9. Prothese nach Anspruch 8, **dadurch gekennzeichnet, dass** die Legierung eine Temperatur am Ende des Auftretens von Austenit (A_{f}) ohne mechanische Spannung aufweist, die unterhalb von 30° C, und vorzugsweise unterhalb von 20° C, liegt.

10. Prothese nach Anspruch 8 oder 9, **dadurch gekennzeichnet, dass** die Legierung aus Titan und aus Nickel besteht, und dass sie 55,7 % ± 0,4 % Nickel umfasst.

## Claims

1. Ossicular prosthesis (14: 114; 214) for the middle ear, of the type adapted to replace at least partially the ossicular chain (3, 6, 10; 103, 106, 110; 203, 206, 210) and to connect a first organ, particularly the stirrup plate or the stirrup, with a second organ, particularly the hammer or the ear-drum, the said prosthesis being capable of automatic elastic self-adjustment, the said prosthesis being formed by an elongate element which includes an arched portion (16; 116; 216) which presents substantially the form of a C, of which the wings are capable of elastic movement towards and away from each other, **characterized in that** the said arched portion is adapted to enter into engagement with at least one of the said organs by an outside surface (16A', 16B'; 116B'; 216B') of at least one (16A, 16B; 116B; 216B) of its wings (16A, 16B; 116A, 116B; 216A, 216B).

2. Prosthesis according to claim 1, **characterized in that** the said arched portion (16) is adapted to enter into engagement with the said first and second organs by means of the outside surface (16a', 16b') of its two wings (16A, 16B).

3. Prosthesis according to claim 1 or 2, **characterized in that** a notch (18), adapted to receive the stud (8) of the stirrup (10), is provided in the said second wing (16A) of the said curved portion (16).

4. Prosthesis according to claim 1, **characterized in that** the said arched portion (116, 216) is adapted to enter into engagement with one of the said organs by the exterior surface (116B', 215B') of a first wing (116Bb; 216B) and is extended, to the level of a second wing (116A; 216A), by a substantially rectilinear portion (122; 222).

5. Prosthesis according to claim 4, **characterized in that** the said rectilinear portion (122; 222) is extended, to its extremity opposite the arched portion (116; 216), by a curved portion (124; 224) adapted to be supported by the stirrup plate.

6. Prosthesis according to any one of claims 1 to 5, **characterized in that** the said arched portion (16) is extended, to the level of a first wing (16B), by a curvaceous portion (20) of which the concavity is directed in opposition to a second wing (16A) of the said arched portion (16).

7. Prosthesis according to any one of claims 1 to 6, **characterized in that** it is made from a metallic alloy which is super-elastic at body temperature.

8. Prosthesis according to claim 7, **characterized in that** the said super-elastic alloy is a shape memory alloy.

9. Prosthesis according to claim 8, **characterized in that** the said alloy possesses in the absence of mechanical constraint a final austenitic appearance temperature (A_{f}) which is less than 30°C and, preferably, less than 20°C.

10. Prosthesis according to claim 8 or 9, **characterized in that** the said alloy is composed of titanium and nickel, and that it contains 55.7% ± 0.4% nickel.
